# EUROPEAN PATENT SPECIFICATION

(11) **EP 4 171 510 B1**
(45) Date of publication and mention of the grant of the patent: **16.10.2024**
(21) Application number: 21754827.0
(22) Date of filing: 24.06.2021
(51) Int. Cl.: A61K 9/00, A61K 9/08, A61K 47/18, A61K 47/36

(54) **OPHTHALMIC COMPOSITION AND USE THEREOF IN THE TREATMENT OF EYE DISEASES**
OPHTHALMISCHE ZUSAMMENSETZUNG UND VERWENDUNG DAVON BEI DER BEHANDLUNG VON AUGENERKRANKUNGEN
COMPOSITION OPHTALMIQU ET SON UTILISATION DANS LE TRAITEMENT DE MALADIES OCULAIRES

(30) Priority: 26.06.2020 IT 202000015457
(43) Date of publication of application: 03.05.2023
(73) Proprietor: SIFI S.p.A., 95025 Aci Sant'Antonio (CT) (IT)
(72) Inventor: ABBATE, Ilenia, 95024 Acireale (IT); CUFFARI, Francesco, 96014 Floridia (IT); SOLFATO, Elena, 95030 Mascalucia (IT); ZAPPULLA, Cristina Maria Concetta, 95127 Catania (IT); CURATOLO, Maria Cristina, 95029 Viagrande (IT); MAZZONE, Maria Grazia, 95025 Aci Sant'Antonio (IT); SPINA, Donato, 95123 Catania (IT); SANTONOCITO, Manuela, 95030 Mascalucia (IT); VIOLA, Santa, 95010 Sant'Alfio (IT); LA ROSA, Luca Rosario, 95045 Misterbianco (IT); GIULIANO, Francesco, 95030 Mascalucia (IT)
(74) Representative: Cattaneo, Elisabetta
(86) International application number: PCT/IB2021/055617
(87) International publication number: WO 2021/260622

(56) References cited:
- EP-A1- 2 002 841
- EP-A1- 3 348 260
- WO-A1-2010/047927
- CN-A- 104 188 874

## Description

### FIELD OF THE INVENTION

The present invention refers to an ophthalmic composition and use thereof in the medical field in the treatment of ocular diseases.

The present invention originates in the sector of pharmaceutical products for ophthalmic use.

In particular, the invention relates to an ocularcomposition for topical use containing natural polymers in combination with at least one osmoprotector.

### BACKGROUND OF THE INVENTION

An increasing number of the population is affected by eye affections and diseases of various etiologies. Among these, one of the most common is dry eye, a multifactorial disease of the ocular surface characterized by a loss of homeostasis of the tear film, and accompanied by ocular symptoms, in which tear film instability and hyperosmolarity, ocular surface inflammation and lesions play etiological roles. Multiple risk factors such as genetic factors, age, sex, diet, environmental conditions, lifestyle, job characteristics, immune status, hormonal status, and medications contribute to altering the morphology and function of ocular surface components (1, 2).

Ocular surface protection is therefore a need felt in many clinical situations, from mild to moderate dry eye to corneal abrasions and ophthalmic surgery outcomes. Furthermore, tear film instability is often accompanied by hyperosmolarity, cell apoptosis, inflammation, and alteration of the epithelial layer of the ocular surface. Reduced production of tears in case of aqueous-deficient dry eye and/or excessive evaporation of the same in the case of lipid deficient or evaporative dry eye lead to tear hyperosmolarity, giving rise to a vicious circle that manifests itself through dry eye signs and symptoms.

A key component of the vicious circle of dry eye is represented by hyperosmolarity, a term used to designate a high increase in electrolytes contained in the tear film, which therefore represents an important goal of the treatment aimed at restoring the homeostasis of the tear film.

Understanding the role of hyperosmolarity caused by tear instability can improve the approach to treatment and the outcomes of dry eye therapies (3, 4).

The effects of hyperosmolarity in dry eye can be directly managed through the use of artificial tears containing compatible solutes with osmoprotective action that help protect the ocular surface cells. The osmoprotective effect, and therefore the regenerative action, of compatible solutes towards the epithelial cell is a function of the time for which these osmoprotective substances can remain in contact with the cells (5, 6).

Ideal characteristics for the protection of the ocular surface are represented by medium viscosity (10-30 cps) and low cytotoxicity (7).

Currently, ocular dryness is often managed with the instillation of ophthalmic solutions, called artificial tears, in the eyes. These solutions may be hypotonictear substitutes. However, artificial tears have a limited persistence on the ocular surface and consequently have only a transient effect on hyperosmolarity.

In recent years, new types of artificial tears contain ing physiologically compatible solutes in association with macromolecules that act as viscosifying elements have been developed.

On the market there are various products, that act as tear substitutes, comprising a polymer having a medium-low concentration, typically in the range of 0.1-0.3%. Most of these products are represented by hyaluronic acid (HA) or cellulose derivatives. However, these prior art products have the drawback of having a low persistence on the ocular surface or an anti-inflammatory and re-epithelialization activity that is not always adequate for the needs.

The patent EP-2002841 discloses an ophthalmic solution for contact lenses comprising Sodium chondroitin sulfate 0.5 g, Sodium chloride 0.55 g,Potassium chloride 0.15 g, Glucose 0. 0025 g Boric acid 0.5 g Borax, Sodium edetate 0.01g, Xanthan gum 0.05 g, Sodium hyaluronate 0.01 g, Hydroxyetinoresorurose 0.1 g, Polyhexanide hydrochloride 0.1 mg, add purified water till Total volume 100 mL (pH 7.0). Additionally amino acids like glycine can be added. The ophthalmic composition of the present invention is useful as an agent for the prophylaxis or treatment of a corneal epithelial disorder, like keratoconjunctivitis, blepharitis or allergic conjunctivitis. The international patent application WO-2010047927 discloses a method useful for treating eyes to improve visual acuity of a person in need of such improvement, the method comprising administering to the person an effective amount of a composition comprising erythritol, glycerol, carnitine, and an aqueous carrier, and a polyanionic component like hyaluronic acid. Additionally a compatible solutes considered to be osmoprotectants can be added and can comprise betaine or glycine. The patent EP-3348260 discloses an aqueous ophthalmic pharmaceutical composition comprising tramadol and a viscosity enhancer chosen from hyaluronic acid, sodium hyaluronate, Xanthan gum and their mixtures. Mannitol as isotonizing agent present. Used for the treatment of ocular pain associated to allergic conjunctivitis, conjunctivitis, blepharitis. The patent CN-104188874B discloses a gentle moisturizing mask containing in percentage by weight: Glycine betaine 2.5%, butanediol 4%, trehalose 1%, hyaluronic acid 0.05%, beta glucan 1.5%; Carbomer 0.15%, xanthan gum 0.05%, and remaining is water.

At present, therefore, the need is felt to have ophthalmic formulations with re-epithelializing, antioxidant and anti-inflammatory activities that are able to restore the physiological homeostasis of the tear film ensuring adequate protection of the ocular surface.

One of the objects of the invention therefore consists in providing an ophthalmic formulation with combined re-epithelizing, tear film osmolarity regulating, anti-inflammatory and anti-oxidant actions.

Another object consists in providing an ophthalmic composition for the treatment and prevention of dry eye having an increased persistence on the ocular surface and increased physical protection.

### SUMMARY

The present invention originates from having found that by combining a linear polymer and a branched polymer with two selected osmoprotectors, a composition with increased re-epithelizing, osmoprotective, anti-oxidant and anti-inflammatory activities and an increase in persistence thereof on the ocular surface is obtained. Ophthalmic compositions have been therefore identified, and form the object of the present invention, wherein two selected osmoprotectors are combined with a linear polymer such as hyaluronic acid and a branched polymer such as xanthan gum.

In the combined compositions of the invention, the polymeric components determine a viscosizing, moisturizing and osmolarity-regulating actions of the tear film; osmoprotectors perform a synergistic action with the polymer matrix in anti-inflammatory and re-epithelialization processes.

According to a first aspect of the invention, an ophthalmic composition comprising a linear polymer based on hyaluronic acid, a branched polymer based on xanthan gum and osmoprotectors, wherein said osmoprotectors comprise a combination of glycine and betaine, optionally in the form of salt such as betaine HCl, is provided. Advantageously, the combined composition as defined herein is for ophthalmicuse. When applied to the surface of the eye, it restores the homeostasis and integrity of the ocular surface system, thus improving symptoms and visual function. The combination of the composition components determines a synergistic re-epithelializing, antioxidant, and protective effects of the external structures of the eye.

According to some embodiments, the compositions object of the invention may include optional buffering, isotonizing and preservative agents, as described in detail below.

The ophthalmic compositions described herein have a surprising ability to act at the level of the ocular surface and therefore allow to carry out, with high efficacy and a wide margin of safety, a targeted topical therapy aimed at preventing or treating diseases in this ocular segment, in particular dry eye. The composition can be used in combination with other products for ophthalmic use treatments for eye diseases.

### DESCRIPTION OF THE FIGURES

Figure 1 A-C illustrate three graphs showing the results of a stability study of Example 2, carried out in three different temperature conditions. Graph A) illustrates the variation of the composition pH over time; graph B) illustrates the variation of the composition osmolarity over time; graph C) illustrates the variation of the composition viscosity over time;
Figure 2 shows a graphical representation showing the rheological profiles of the two polymers, sodium hyaluronate (NaHa) and xanthan gum (XNT) with respect to the combination thereof (Formulation);
Figure 3 A-C illustrate three bar graph representations showing, respectively, gene expression levels of A) COX2, B) TNFA, and C) ILB in SIRC cells subjected to hyperosmolar stress with 500 mOsm NaCl and treated for 4h with: 0.1% glycine (GLY), 0.07% proline (PRO), 0.25% betaine (BET), a 0.1% GLY + 0.25% BET mixture, and a 0.1% GLY + 0.07% PRO mixture;
Figure4 shows a bar graph illustrating the scratch assay results for 0.2% xanthan (0.2% XNT), 0.2% sodium hyaluronate (0.2% HA) and a 0.2% xanthan + 0.2% hyaluronate mixture (0.2% XNT + 0.2% HA) on HCE cells;
Figure 5 shows a bar graph illustrating the antioxidant effect of 0.2% xanthan (0.2% XNT), 0.2% xanthan + 0.2% sodium hyaluronate mixture (0.2% XNT + 0.2% HA) and combination thereof with the osmoprotectors 0.1 % glycine and 0.25% betaine (XNT + HA + Gly + Bet) according to the invention, on HCE cells;
Figure 6 illustrates a bar graph showing COX2 gene expression levels in A549 cells pre-treated for 2h with 0.2% xanthan (XNT), 0.2% sodium hyaluronate (HA) and a 0.2% XNT + 0.2% HA mixture and induced with IL1B for 18h.
Figure 7 schematically illustrates the three-dimensional lattice interaction generated between the polymer chains of xanthan gum and hyaluronic acid.
Figure 8 illustrates photographic microscopic reproductions of 3D multilayers of HCE cells subjected to the scratch assay model to detect the re-epithelialization activity of the four-component composition of the invention with respect to the single components or combinations of two or three of the same, as described in Example8.
Figure 9 illustrates bar graphs showing cell viability test results by lactate dehydrogenase (LDH) assay after oxidative stress induced by glucose oxidase (GOX). The data illustrate the antioxidant effect of the individual components, 0.1% glycine, 0.007% proline, 0.25% betaine, versus their associations such as (GLY + BET) and (GLY + PRO). The data illustrated in this Figure are also to be intended as precursors of EXAMPLE 7, relating to the anti-oxidant activity on HCE cells.
Figure 10 illustrates graphs relating to the results of comparative stress tests at 60°C between the composition F1 of Example 2 with 4% mannitol and the composition E1, known in the art, as described in Example10.

### DETAILED DESCRIPTION

A composition comprising a combination of the polymers hyaluronic acid, or a pharmaceutically acceptable salt thereof, and xanthan gum with the specific osmoprotectors glycineand betaine is described herein. The combined composition of the invention is suitable for use in the ophthalmic field, in particular in the treatment of dry eye disease.

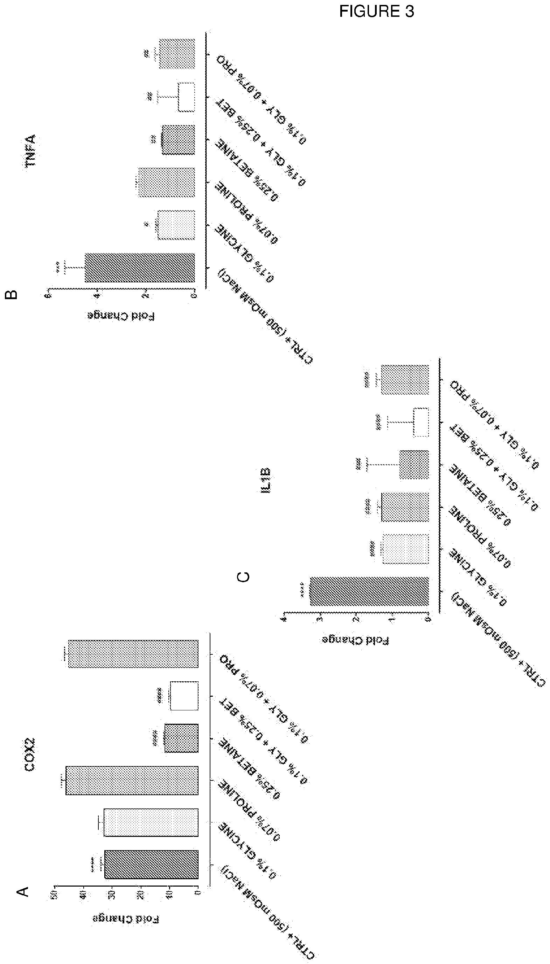

In particular, it was observed that the combination of the two polymeric components with the selected amino acid osmoprotectors glycine and betaine results in a surprising increase in the speed of re-epithelialization and healing of lesions of the external structures of the eye. In particular, the authors observed how in the composition, the combination of the two components glycine (Gly) and betaine (Bet) results in an unexpected osmoprotective effect in the eye. This technical effect, found experimentally in an experimental dry eye model according to Example 4, is highlighted in Figure 3.

The combined composition of the invention has moisturizing and lubricating properties for the ocular surface and is provided with a re-epithelialization and anti-oxidant actions. Furthermore, when instilled in the eye, it restores the physiological osmolarity of the tear film, particularly in subjects suffering from dry eye syndrome. Advantageously, the composition of the invention comprises two physiologically acceptable polymers, a linear polymer such as optionally salified hyaluronic acid and a branched polymer such as xanthan gum.

The object of the present invention isan ophthalmic composition as defined in claim 1.

Further embodiments of the composition are defined in the attached dependent claims 2-9.

Preferably, the composition of the invention does not contain boric acid and/or sodium borate hydrate, also known as borax. The presence of one or both of these ingredients is generally not suitable for the ophthalmic use described herein, as it is poorly tolerated for ophthalmic use.

### Combined Composition

A polymeric component of the composition is hyaluronic acid, a glycosaminoglycan containing alternating units of D-glucuronic acid (GlcUA) and N-acetyl-D-glucosamine (GlcNAc) linked via alternating β-1,4 and β-1,3 glycosidic bonds. Hyaluronicacid is biocompatible as it is naturally present in various human tissues, such as umbilical cord, vitreous humor, and the extracellular matrix of soft connective tissues. The physico-chemical characteristics of hyaluronic acid depend in part on its molecular weight and interactions with CD44 receptors present in most of the cells on the ocular surface. One of its main features is the hygroscopic capacity which allows it to retain an amount of water up to 1,000 times its weight. It also has a viscoelastic rheological behavior, which allows it to provide relief by reducing friction during blinking and eye movements.

Hyaluronic acid can be either as such or in a salt form, for example as sodium hyaluronate.

According to some embodiments, the composition contains hyaluronic acid or a physiologically acceptable salt thereof in an amount comprised in the range from 0.01 to 5%, from 0.05 to 0.5%, from 0.1 to 0.3% weight/volume (g/100ml).

Another polymeric component of the composition is xanthan gum, an anionic branched polysaccharide produced by natural aerobicfermentation, typically of corn starch, with bacteria, such as *Xanthomonas campestris.*

For example, a suitablexanthan gum has an average molecularweightrangingfrom 3 to 7.5 MDa.

Typically, it is a branched polysaccharide based on repeating units represented by D-glucose, D-mannose, and D-glucuronic acid, in a molar ratio of 2:2:1, and by a variable component represented by O-acetyl and pyruvic residues.

It is a highly hydrophilicviscosizing polymer, capable of binding large amounts of water in a reversible way. It is able to adhere to the mucin layer of the corneal epithelium through the formation of non-covalent bonds. It has lubricating and anti-oxidant properties, and acts as a re-epithelializing agent scaffold. Its pseu doplasticity (non-Newtonian behavior) contributes to the maintenance of a high initial viscosity and a progressive viscosity reduction during eyelid movements, thus minimizing the friction between the eyelids and the ocular surface and improving patient comfort.

According to some embodiments, the hyaluronic acid is a linear polymer and advantageously can have an average molecularweight in the range from 0.2 MDa to 2.5 MDa, preferably from 0.5 to 2.0 MDa.

Hyaluronic acid molecular weights are expressed as average molecular weight (MW) (Da). For example, the average molecular weight of hyaluronic acid and possibly of the polymers mentioned in this description can be determined using standard methods in the field, such as those described by Ueno et al., 1988, Chem Pharm Bull. 36, 4971-4975; Wyatt 1993, Anal Chim Acta 272: 1-40; Watt Technologies 1999 "Light scattering University Dawn Course Manual and "Dawn Eos Manual" Wyatt Technology Corp. Santa Barbara CA (USA).

According to some embodiments, hyaluronic acid is in the form of a salt with an alkaline or alkaline earth metal, both pharmaceutically acceptable, for example sodium, potassium, magnesium, or calcium hyaluronate and the like.

According to some embodiments, the composition contains xanthan gum or a derivative thereof in an amount comprised in the range from 0.01 to 5%, from 0.05 to 0.5%, from 0.1 to 0.3% weight/volume (g/100 ml).

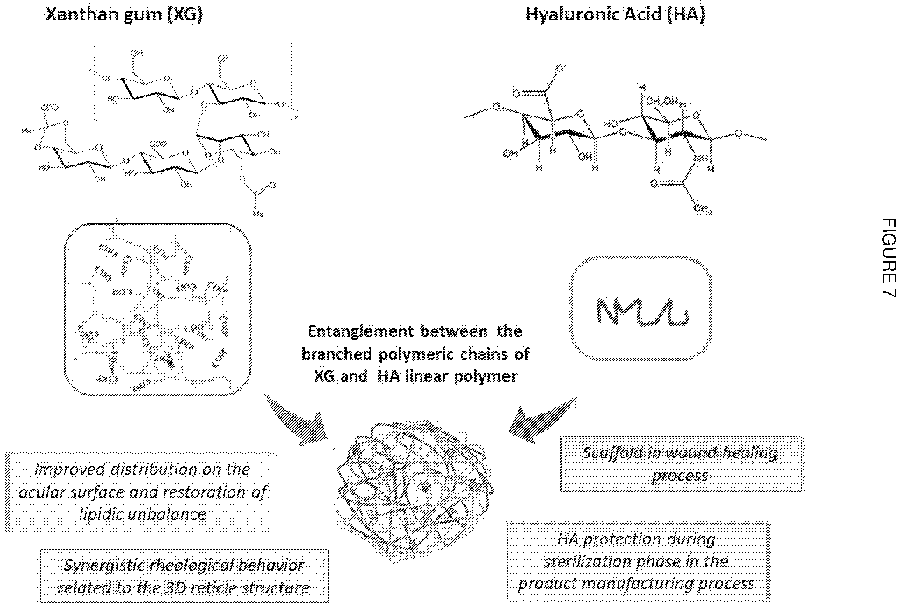

It has been observed that the combination between the linear polymer, hyaluronic acid, and the branched polymer, xanthan gum, gives rise to a chemical-physical interaction, i.e. the formation of peculiar entanglements that indicate the physical interconnection between the two polymers. This interaction provides hyaluronicacid with physical protection during the sterilization phase in the production process. The formation of a three-dimensional lattice, as illustrated in Figure 7, also provides the formulation with specific properties such as: scaffold in ocularre-epithelialization processes; synergy in the rheological behavior of the two polymers associated with the physical interaction of the same; an improvement in the oculardistribution of the tear film lipid layer thanks to the presence of a substrate on which lipids themselves can be reorganized.

Furthermore, according to an aspect of the invention, the composition can contain or incorporate in the meshes of said three-dimensional lattice, a pharmacologically active principle and convey it to the surface of the eye following instillation or local application.

According to this aspect, the composition is a carrier of pharmacologically active principles, as described below.

The composition of the invention includes osmoprotectors, wherein said osmoprotectors comprise a combination of glycine and betaine, optionally in form of salt preferably as betaine HCl.Osmoprotectors are typically natural organic molecules, compatible solutes or organic osmolytes that act by counteracting damage from hyperosmolarstress. The cells of the ocularsurfaceare ableto absorb these compatible solutes, which thus contribute to restoring cell volume, stabilizing protein function and restoring the functioning of cellular processes.

Advantageously, the combination of osmoprotectors of the composition regulates tear osmolarity within physiological values. This action is particularly useful in eye diseases in which there is a tear hyperosmolarity resulting, for example, from an increase in the concentration of electrolytes in the tear fluid. The osmoprotectors belong to one of the following classes: amino acids preferably betaine, taurine, glycine, proline, L-carnitine; polyols for example, glycerol, erythrol, xylitol, mono-inositol; saccharides, for example trehalose. The composition of the invention comprises the osmoprotectors glycine and betaine. Glycine belongs to the class of proteinogenic essential amino acids, which are able to provide a fundamental contribution in the epithelial remodeling and collagen biosynthesis processes.

The term betaine means a neutral compound with a positively charged cationic functional group, such as a quaternary ammonium, NH4+, where one or more hydrogen atoms can be generic functional groups, or a phosphonium cation, PR1R2R3R4+, where R1, R2, R3 and R4 are hydrogen atoms or still generic functional groups, which do not directly bind to hydrogen, but to a negatively charged functional group, such as a carboxylate anion, chloride, hydroxide or tartrate, which is not proximal to the cationic site.

Preferably the betaine is N,N,N-trimethylglycine or glycine betaine.

According to some embodiments, the composition further comprises at least one isotonizing agent. The term isotonizing agent or isotonizer refers to a compound or substance that regulates the tonicity of an ophthalmic liquid.

Examples comprise alkali metal salts such as sodium chloride, potassium chloride or mannitol, glycerin, propylene glycol, dextrose, ethylene glycol. The amount of tonicity agent may vary depending on the specific contribution. According to some embodiments, the osmotic agent maintains the osmolarity of the combination composition at a value of ≤250 mOsmol/Kg.

Ophthalmic composition with this hypotonia statistically improve the symptoms and condition of the ocular surface of patients suffering from dry eye syndrome.

The combined composition may further include a buffer, or buffering system, i.e. any weak conjugate acid-base pair suitable for maintaining a desirable pH range. Suitable buffers include, but are not limited to, acetate buffers, citrate buffers, phosphate buffers, borate buffers, or a combination thereof, such as citrate/phosphate, or still trometamol, histidine or arginine buffers.

For example, the composition of the invention may contain a buffer or buffering system comprising sodium hydrogen phosphate, in particular monohydrate, disodium phosphate, in particular dodecahydrate.

Anothersuitable buffer comprises sodium citrate.

Acids or bases may be used to adjust the pH of these formulations as needed, and the amountof buffer used can vary.

According to a preferred embodiment of the present invention, the buffering agent is trometamol, and the pH of the formulation is comprised in the range from 4.0 to 9.0, preferably from 6.6 to 7.8, specifically from 6.8 at 7.6.

Such a buffer can have a concentration in a range from 10 to 50 mM, and the pH in the buffered solution thereof can be increased by addition of sodium hydroxide or another base, or reduced by addition of hydrochloric acid or another acid.

In some embodiments the composition object of the present invention is free of preservatives.

Accordingto some embodiments, the composition comprises at least one isotonizer selected from magnesium chloride, calcium chloride and mixtures thereof. In some embodiments, the isotonizer includes magnesium chloride and calcium chloride.

Preferably the magnesium chloride is magnesium chloride hexahydrate, and the calcium chloride is dihydrate.

According to some embodiments, the composition of the invention comprises a further glycerol-based isotonizer.

Preferably, the composition of the invention has a pH comprised in the range from 4.0 to 9.0, preferably from 6.8 to 7.6, adjusted by adding buffers and isotonizers as described herein.

Advantageously, the composition of the invention is a sterile, preferably hypotonic, ocular composition.

Preferably the composition is hypotonicwith respect to the tear fluid and has a value comprised in the range from 200 to 270mOsm/Kg.

Preferably, the composition of the invention is in liquid form, for example as an aqueous solution, or in a semi-liquidform as a gel. Advantageously, the composition is an eye drop to be instilled in the eye or an ophthalmic gel to be applied onto the eye.

Preferably, the compositions of the invention do not contain preservatives and/or antimicrobials or in any case they have a trace content lowerthan 0.001 % (w/v). Compositions of the invention formulated in multidose may also contain antimicrobial preservatives such as, for example, parabens, quaternary ammonium salts, polyhexamethylene biguanide (PHMB) and others possibly included among those usable in the compositions for ophthalmic use.

The solventused in the compositions in liquidformis preferably wateror an aqueous solution of one or more components compatible with topical ophthalmic use. Typically, the composition may further include emulsifying, stabilizing, solubilizing, antioxidant agentsof both natural and synthetic origin, and mixtures thereof.

### Medical Uses

According to another aspect, a composition as defined in claim 1 or in any of the dependent claims for use as a medicament is provided.

The composition for medical use finds application in preventing and/or treating eye diseases.

A further object of the invention is therefore an ophthalmic composition as described herein for use in the treatment of inflammatory eye diseases, lesions or abrasions of the ocular surface, hyperosmolarity of the tear film. In one aspect, the ophthalmic composition described herein is for use in the treatment of a disease or inflammatory states of the ocular surface.

According to one aspect, an ophthalmic composition as described herein for use in the treatment of dry eye is provided.

Advantageously, the composition of the invention can deliver pharmacologically active ingredients in the eye to prevent and/or treat eye diseases.

According to some embodiments, the composition therefore contains a pharmacologically active ingredient preferably selected from antihistamines, antibiotics, anti-glaucomatous drugs, for example beta blockers, prostaglandin inhibitors, carbonic anhydrase inhibitors, alpha adrenergic agonists, steroidal and non-steroidal anti-inflammatories, antiallergics, antivirals, antiseptics, anesthetics, mydriatics, lipid molecules with anti-inflammatory and/or anti-oxidant activity, bacterial fermentation products, for example pre-, post-biotic products, immunomodulators, hormones, vitamins, animal secretion proteins such as lactoferrin , colostrum, and mixtures thereof.

According to another aspect, a composition as described above, comprising a pharmacologically active principle selected from antihistamines, antibiotics, anti-glaucomatous drugs, steroidal and non-steroidal anti-inflammatory drugs, antiallergics, antivirals, antiseptics, anesthetics, mydriatics, lipid moleculeswith anti-inflammatory and/or anti-oxidant activity, bacterial fermentation products, for example, pre-, post-biotic products, immunomodulators, hormones, vitamins, animal secretion proteins such as lactoferrin, colostrum, and mixtures thereof, for use in the treatment of an eye affection or disease is provided. According to this latter aspect, the composition of the invention finds application in the topical treatment of allergic syndromes, infectious and inflammatory corneal-conjunctival diseases of bacterial or viral origin such as conjunctivitis, blepharitis and keratitis, glaucoma, dry eye with mild-moderate to severe symptoms, and in prevention in cases of ophthalmic surgery.

According to some embodiments, the ophthalmic composition described herein contains bromfenac as an active principle. According to this embodiment, the ophthalmic composition is for use in the treatment of inflammatory diseases both of the ocular surface, in particular of external anatomical structures such as conjunctiva and cornea, and of the back of the eye.

The compositions described herein can be in the form of single-dose or multidose medicament.

For administration purposes, the above compositions can be made as eye drops or in liquid orgel form.

The following examples further illustrate the invention without constituting a limitation thereof.

### EXAMPLE 1

Composition for application or instillation in the eyes having the following formulation:

| | |
|---|---|
| Xanthan gum, | 0.200% (w/v) |
| Sodium hyaluronate, | 0.200% (w/v) |
| Glycine, | 0.100% (w/v) |
| Betaine HCl, | 0.250% (w/v) |
| Tris base, | 0.485% (w/v) |
| Sodium chloride, | 0.400% (w/v) |

### EXAMPLE 2

Composition for application or instillation in the eyes having the following formulation:

| | w/v | Range (w/v) |
|---|---|---|
| Bromfenac* | 0.090%(w/v) | |
| Xanthan gum, | 0.200% (w/v) | |
| Sodium hyaluronate, | 0.200% (w/v) | |
| Glycine, | 0.100% (w/v) | |
| Betaine HCl, | 0.250% (w/v) | |
| Tris base, | 0.485% (w/v) | 0.2%-0.6% |
| Sodium chloride, | - | 0,1%-0.5% |
| Sodium Citrate x 2H2O | 0.290%(w/v) | 0.1 %-2.0% |
| Mannitol | 2.000%(w/v) | 1.0%-4.0% |
| EDTA | - | 0.05%-2.0% |
| *Equivalent to Bromfenac sodium sesquihydrate | 0.1035% | |

### EXAMPLE 3

The composition of Example 1 was subjected to a stability study in order to evaluate the chemical-physical and microbiological characteristics, according to the conditions reported in the ICH Q1A(R2) Stability Testing Guidelines: stability testing of new drug substances and products.

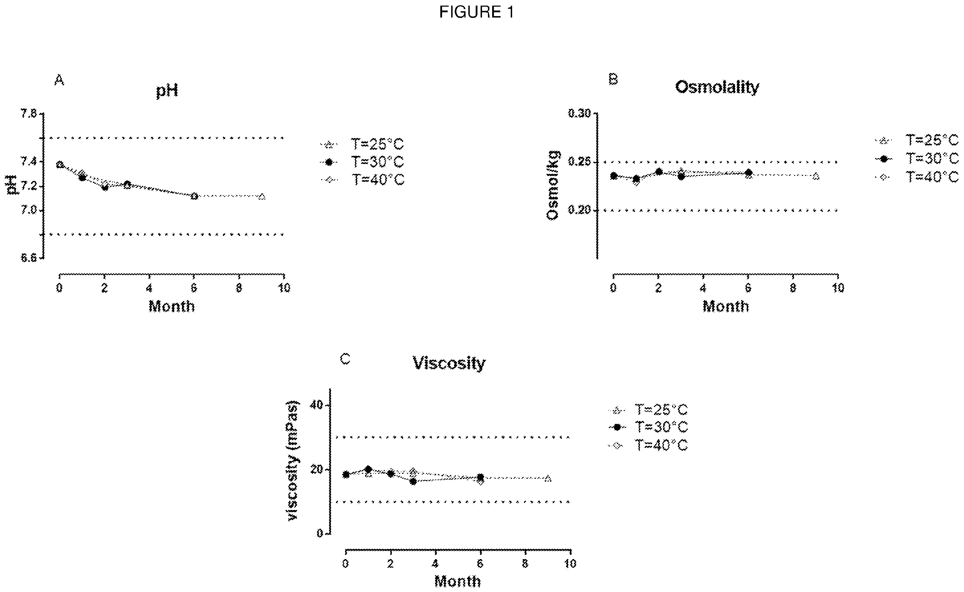

Figure 1 shows the data relating to the study conducted both in *long term storage conditions* (25°C±2°C, and 60%RH±5%), and *intermediate conditions* (30°C±2°C, and 65%RH±5%) and *accelerated storage conditions* (40°C±2°C, and 75%RH±5%). The experimental data show a high stability of the chemical-physical properties of the formulation tested, in all study conditions, including the *accelerated storage conditions* which are predictive of stability at 24 months in the *long term conditions.*

### EXAMPLE 4

A test was carried out to evaluate the rheological behavior of the two polymers, i.e. hyaluronic acid and xanthan gum, contained in the composition of Example 1. The rheological profile was evaluated both at low shear stress values (simulation of the instillation time in the eye of the composition of Example 1) and at high shear stresses (simulation of the eyelid blinking phenomenon).

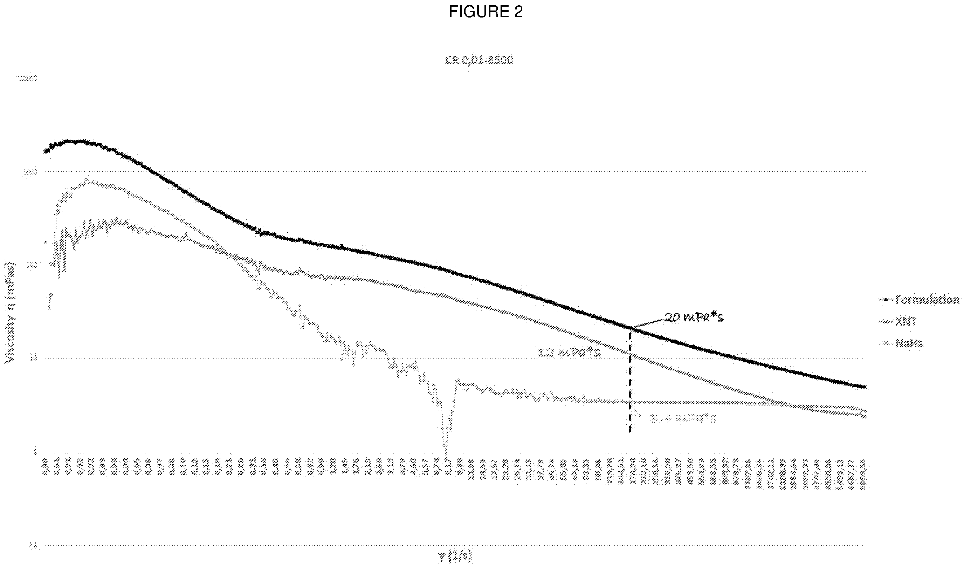

The data showed a higherviscosity value for the combination of the two polymers compared to the individual hyaluronic acid and xanthan gum polymers. This behavior is highlighted by the graph shown in the attached Figure2.

High viscosity values at low shear rates give evidence of a longer residence time on the ocular surface, while a lower viscosity at high shear rates indicate better patient compliance.

At the shear stress value of γ= 150 sec⁻¹, the viscosity of the individual sodium hyaluronate (HA) and xanthan gum (XNT) solutions is of 3.4 and 12 mPas, respectively. The solution containing the combination of the two polymers produced a viscosity of 20 mPas, which was 77% higher than expected from a simple combination. These data highlight and support the synergistic effect of the combination of the two polymers.

### EXAMPLE 5

### Osmoprotective Effect in an in vitro Dry Eye Model

A test was carried out to evaluate the potential protective effect of the osmoprotectors glycine, proline and betaine alone or in a mixture of two (Glycine + Betaine; Glycine + Proline).

In an *in vitro* Dry Eye Model, hyperosmolar stress was induced with NaCl (500 mOsm) in rabbit corneal epithelial cells (SIRC). The protective effect was evaluated by analyzing the expression of pro-inflammatory genes such as cyclo-oxygenase 2 (COX2), tumor necrosis factor A (TNFA) and interleukin 1B (ILB).

Real Time RT-PCR data showed that treatment with 0.1% glycine(GLY) and 0.07% proline (PRO) reduced the expression of messengers of TNFA (Fig. 3B) and IL1B (Fig. 3 C) with respect to CTRL + (NaCl 500 mOsm), although proline did not reach statistical significance againstTNFA, as illustrated in the attached Figure 3 B.

The mixture of 0.1 % GLY + 0.07% PRO was able to reduce both pro-inflammatory genes (TNFA, ^{##}p≤0.001%; IL1B, ^{####}p≤0.0001%) with respect to CTRL+, even if the effect seems attributable to the action of glycine alone, rather than to a synergy of both amino acids (Figures 3 B and 3 C).

Treatment with 0.25% betaine (BET) was shown to be effective in reducing the expression of all three pro-inflammatory genes analyzed by about 70% with respect to CTRL + (Figures 3 A-C).

Unlike the mixture of 0.1 % GLY + 0.07%PRO, it was observed how the treatment of SIRC with the combination of 0.1% GLY + 0.25% BET (combination of polymers contained in the composition of the invention) reduced significantly the expression levels of COX2, TNFA and IL1B with respect to CTRL + with greater efficacy than treatment with single osmoprotectors, thus showing a synergistic action of the glycine and betaine combination (Fig. 3 A-C).

These results demonstrate that the combination of the osmoprotectors glycine and betaine, contained in the combined composition of the invention, have a high osmoprotective effect, showing a synergistic effect in reducing the expression of genes involved in inflammatory processes that are triggered under hyperosmotic stress conditions.

The data are summarized in the graphs of Figure 3 showing the gene expression levels of A) COX2, B) TNFA and C) ILB in SIRC cells subjected to hyperosmolar stress with 500 mOsm NaCl and treated with 0.1% GLY, 0.07% PRO, 0.25% BET, 0.1% GLY + 0.25% BET or 0.1% GLY + 0.07% PRO for 4h. The data represent the mean ± SEM of the Fold Chance of n = 3 replicates per condition with respect to the calibrator (CTRL-, 300 mOsm).

^{#}p≤0.05; ^{##}p≤0.01; ^{###}p≤0.001; ^{####}p≤0.0001 vs. CTRL+ (500 mOsm); ***p≤0.001; ****p≤0.0001 vs. CTRL-. One-way ANOVA followed by Sidak's *post-hoc* test.

### EXAMPLE6

### Re-epithelialization activity on monolayer human corneal epithelial (HCE)cells

A test was carried out to evaluate the re-epithelialization activity of 0.2% xanthan (0.2% XNT) and 0.2% hyaluronicacid(0.2% HA) polymers alone and in combination (0.2 % XNT + 0.2% HA) on human corneal epithelial (HCE) cells by scratch assay. The analysis of the scratch areas shows that, at T24h, 0.2% XNT and 0.2% HA, when used alone are not able to reduce the scratch areas in a statistically significant way with respect to the basal condition (Basal).

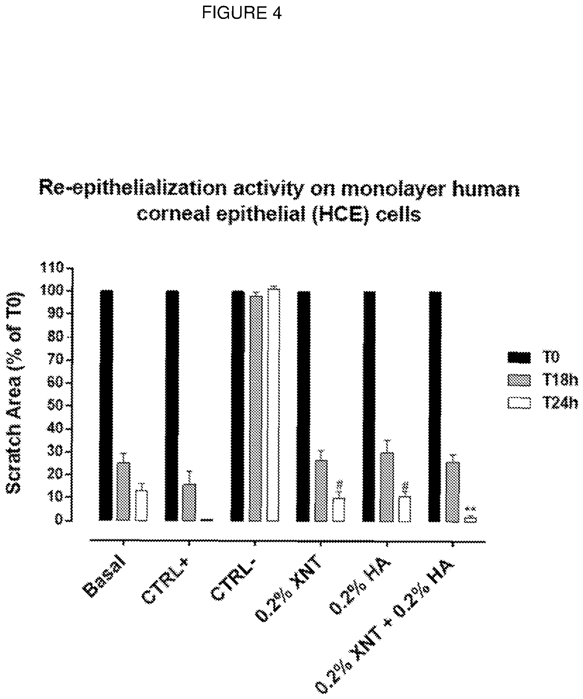

Conversely, the combination of 0.2% XNT + 0.2% HA significantly reduces the scratch area at T24h with respect to Basal showing a synergistic effect (**p≤0.01 vs. Basal, Fig. 4). Furthermore, the statistical analysis shows that 0.2% XNT and 0.2% HA alone are statistically different from the 0.2% XNT + 0.2% HA association (^{#}p≤0.01 vs. 0.2% XNT + 0.2% HA, Fig. 4) further supporting the synergistic effect of the two polymers.

The data of the tests are summarized in Figure 4 showing the closure of the scratch area trend from T0 to T24h in the presence of 0.2% XNT, 0.2% HA and the 0.2% XNT + 0.2% HA association on HCE cells. **p≤0.01 vs. Basal. ^{#}p≤0.05 vs. 0.2% XNT + 0.2% HA. Statistical analyzes were carried out using the One way ANOVA test followed by Sidak's *post-hoc* test.

### EXAMPLE 7

### Anti-oxidant activity on HCE cells

A test was carried out to verify the anti-oxidant effect of 0.2% xanthan (XNT 0.2%), 0.2% xanthan + 0.2% hyaluronic acid (0.2% XNT + 0.2% HA) and the association thereof with the osmoprotectors 0.1% glycine and 0.25% betaine (XNT + HA + Gly + Bet) on HCE cells.

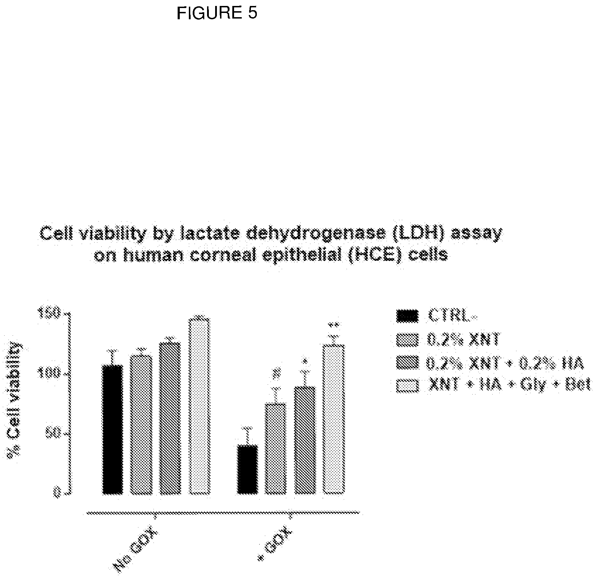

Oxidative stress was induced with glucose oxidase (GOX) and the antioxidanteffect, in terms of cell viability, was measured with the lactate dehydrogenase (LDH) assay. From the analysis of the percentages of cell viability reduction, it is observed that 0.2% XNT alone leads to an increase in cell viability which, however, does not reach statistical significance with respect to CTRL- (74% viability, Fig. 5). Conversely, the association of 0.2% XNT + 0.2% HA increases cell viability in a statistically significant way with respect to CTRL- (88% viability, *p≤0.05, Fig. 5). Furthermore, it is interesting to note that this effect becomes even more evident when the two polymers are combined with osmoprotectors in the XNT + HA+ Gly + Bet mixtures. In fact, the XNT + HA + Gly + Bet association increases cell viability (123% viability) in a statistically significantway both with respect to CTRL- (**p≤0.01) and to 0.2% XNT (^{#}p≤0.05 Fig. 5).

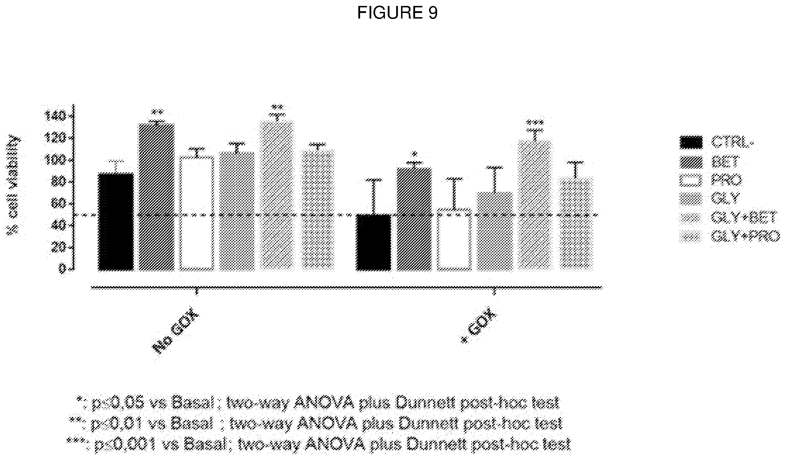

The results obtained highlighted the anti-oxidant effect of the two polymers, 0.2% xanthan and 0.2% HA, when used in association. Furthermore, it is worth noting that this effect is even more evident when the two polymers are in association with the osmoprotectors 0.1% glycine and 0.25% betaine showing a surprising and advantageous synergistic effect of the components. The cell viability test using the lactate dehydrogenase (LDH) assay after oxidative stress induced with glucose oxidase (GOX), was also carried out on the individual osmoprotectors and on the association of these latter as shown in Fig. 9.

Individual osmoprotectors 0.25% betaine and 0.1% glycine, show a marked antioxidanteffectperse (Fig. 9): this effect was highlighted by the LDH assay where cell viability measured after treatment with GOX is equal to 65% for glycine and 98% for betaine. The association of both osmoprotectors (GLY + BET) showed a higher antioxidant effect (108%, ***p≤0.001 vs Basal, Fig. 9) with respect to the individual components and with respect to the (GLY + PRO) association. These data confirm the synergy of their association which provides a not negligible, and at the same time advantageous, contribution to the association with the polymers within said formulation (see Fig. 5).

### EXAMPLE 8

### Anti-inflammatory activity on human lung adenocarcinoma cells (A549)

In order to evaluate the potential anti-inflammatory effect of 0.2% XNT and 0.2% HA polymers both as single active principles and as a mixture, the *in vitro* assay system *"IL-1 beta-induced COX-2 expression*/*activity"* was used on A549 cells.

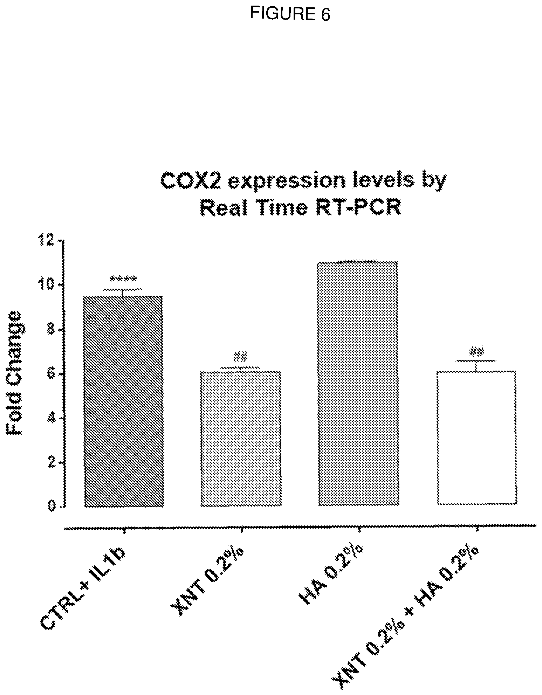

Of the two polymers tested, only the treatment with 0.2% XNT was able to significantly reduce (##p≤0.01%) the expression of COX2 with respect to CTRL+IL1B (Fig. 6).

Furthermore, it was observed that the treatment of A549 with the 0.2% XNT + 0.2% HA combination was able to significantly reduce (^{##}p≤0.01%) the expression of the analyzed pro-inflammatory gene with respect to CTRL+IL1B.

Data are summarized in Figure 6 illustrating COX2 gene expression levels in A549 cells pre-treated for 2h with 0.2% XNT, 0.2% HA or 0.2% XNT + 0.2% HA and induced with IL1B for 18h. The data represent the mean ± SEM of the Fold Change of n = 3 replicates per condition with respect to the calibrator (CTRL-).

##p≤0.01 vs. CTRL+IL1B; ****p≤0.0001 vs. CTRL-. One-way ANOVA followed by Sidak's *post-hoc* test.

### EXAMPLE 9

### Comparative test on re-epithelialization activity on multilayerHCE cells.

An *in vitro* re-epithelialization comparative study was carried out on a 3D multilayer of HCE cells, subjected to the scratch assay model, to find the type of activity obtained from the combination of the composition components: hyaluronic acid, in particular the sodium salt, xanthan gum, glycine and betaine, in particular betaine HCl.

The activities of the following components/ingredients were compared:
- Hyaluronic acid (HA) and trehalose mixture
- Hyaluronic acid (HA)
- Xanthan gum
- Hyaluronic acid and xanthan gum (XNT)
- Glycine and betaine
- Hyaluronic acid sodium salt, xanthan gum, glycine, and betaine HC.

In order to monitor the different phases of the corneal re-epithelialization process (migration and remodeling phases) at 24 and 48 hours after wound induction, the following investigations were carried out:
- Histological analysis with hematoxylin/eosin (H&E);
- Immunohistochemical analysis on integrin β1 (ITGβ1)
- Metalloproteinase 9 (MMP9)

These are elements and processes involved in the modulation of the migration phase and in the remodeling of the extracellular matrix (ECM) during the re-epithelialization process.

The results of the comparative tests at 24 and 48 hours, respectively, are summarized in the following Comparative Table:

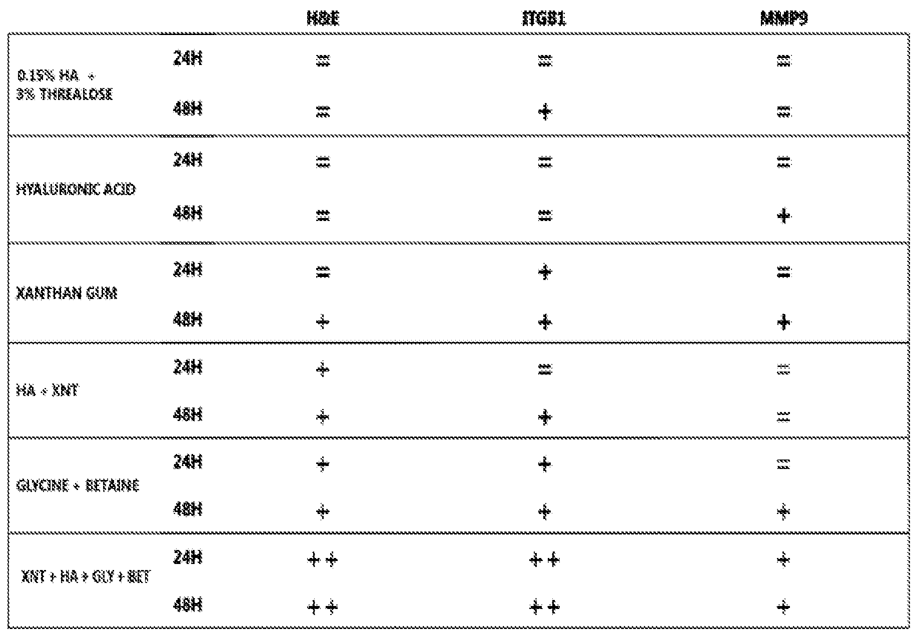

As it can be seen from the above results, the formulation containing hyaluronic acid sodium salt, xanthan gum, glycine, and betaine HCl according to the invention showed the best results, promoting the re-epithelialization process from the beginning of the migratory phase and allowing both wound closure and effective remodeling of the ECM as early as 24 hours. It has been observed that the re-epithelialization effect with the composition of the invention issurprisinglyfasterthan the effect found with the other components tested.

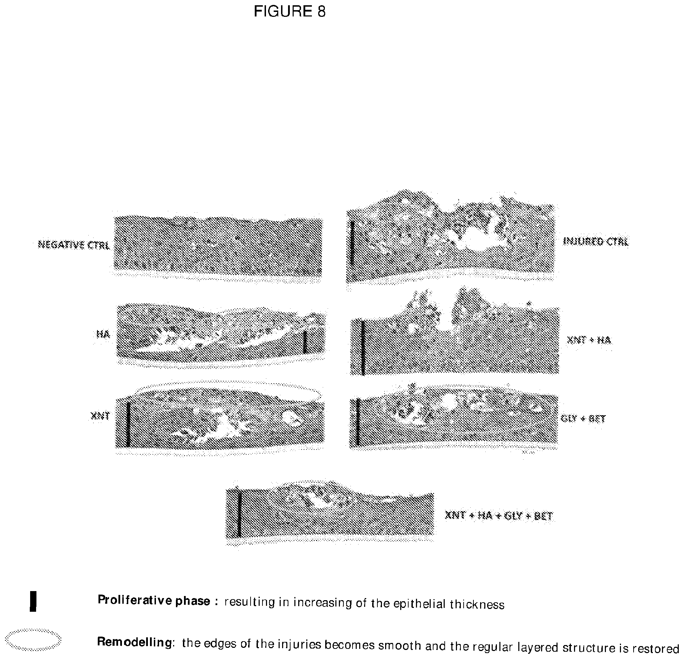

The results found are immediately apparent from the photographic reproductions illustrated in the attached Figure 8.

Conclusions from the comparative test:
- 0.2% Xanthan gum: At 48h it favored an efficientmodulation of the migration phase
- 0.2% Hyaluronic acid: At 48h it promotes tissue remodeling
- 0.2% Hyaluronic acid + 0.2% Xanthan Gum: At 24h the synergistic effect favored early cell proliferation
- 0.1% Glycine + 0.25% Betaine: At 24h the two-component mix showed early efficacy in promoting the migration phase and anticipating tissue remodeling
- 4-Ingredient combination composition of the invention: it showed the best results by promoting the re-epithelialization process from the beginning of the migratory phase, favoring wound closure and efficient remodeling of the extracellular matrix (ECM).

### EXAMPLE 10

### Stress test stability study at 60°C at 4 weeks.

A composition of Example 2 was subjected to a stress test study, undertemperature conditions of 60°C, to evaluate the performance and the chemical-physical characteristics of the formulation. The experimental data show a high stability of the chemical-physical properties of the tested formulation.

In particular, a composition F1 having the formulation of Example2 and an amount of mannitol of 4% was tested.

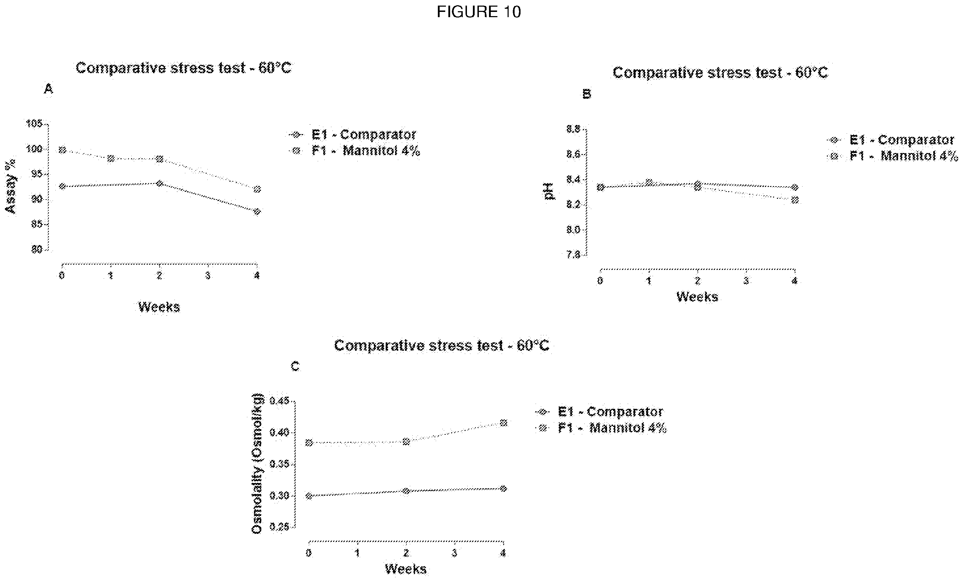

This composition of the invention was subjected to a comparative stress test study, under temperature conditions of 60°C, and compared with a formulation with a different composition currently on the market, as shown in Table 1 below (Fig.10-E1).

**Table 1. Composition Formulation E1**

| Components | Function |
|---|---|
| Bromfenac | Active - 0.090%(w/v) |
| Sodium tetraborate (Borax) | Buffer |
| Anhydrous sodium sulfite | Isotonizing agent |
| Tyloxapol | Stabilizing agent |
| Povidone (K30) | Stabilizing agent |
| Benzalkonium chloride | Antimicrobial agent |
| Disodium edetate | Chelating agent |
| Sodium hydroxide | pH Corrector |
| Water | Solvent |

In particular, at 4 weeks at 60°C both formulations show a constant pH trend throughout the study period, but the assay percentage (Assay%) of the amount of Bromfenac in the formulations tested during the stability study highlights greater stability for the formulation F1, when compared to the formulation E1, i.e. 92% and 87%, respectively.

The composition of the formulation object of the present application has unexpectedly led to better stability results for the chemical-physical parameters taken into consideration, in particular for the recovery of the active Bromfenac assay, as shown by the graphs in the attached Fig. 10.

## Claims

1. An ophthalmic composition comprising a linear polymer based on hyaluronic acid or a pharmaceutically acceptable salt thereof, a branched polymer based on xanthan gum or a pharmaceutically acceptable salt thereof, and osmoprotectors, wherein said osmoprotectors comprise a combination of glycine and betaine, optionally in form of salt preferably as betaine HCl.

2. The composition according to claim 1, wherein hyaluronic acid or a salt thereof is present in an amount comprised in the range from 0.01 to 5%, from 0.05 to 0.5%, from 0.1 to 0.3 w/v, and the xanthan gum or a salt thereof is comprised in the range from 0.01 to 5%, from 0.05 to 0.5, from 0.1 to 0.3% w/v.

3. The composition according to claim 1 or 2, wherein glycine is present in an amountcomprised in the rangefrom 0.01 to 1%, from 0.05 to 0.2% w/v and/or betaine is present in an amountcomprised in the rangefrom 0.01 to 3%, from 0.1 to 0.5% w/v and optionally the hyaluronic acid has an average molecular weight in the range from 0.2 MDa to 2.5 MDa.

4. The composition according to any one of claims 1-3, wherein the hyaluronic acid is in the form of a pharmaceutically acceptable salt selected from sodium, potassium, calcium, magnesium, and cationic amino acids.

5. The composition according to any one of claims 1-4, further comprising an isotonizing agent preferably selected from sodium chloride, potassium chloride, mannitol, glycerin, dextrose, glycol such as for example propylene or ethylene glycol and mixtures thereof.

6. The composition according to any one of claims 1-5, further comprising a buffer or a buffer system preferably selected from acetate buffers, citrate buffers, phosphate buffers, borate buffers, trometamol-based buffers, histidine, arginine, and buffer or buffer system mixtures, emulsifying agents, stabilizers, solubilizers, anti-oxidants, and mixtures thereof.

7. The composition according to any one of claims 1-6, having a pH from 4.0 to 9.0, preferably from 6.8 to 7.6.

8. The composition according to any one of claims 1-7, in liquid form as an aqueous solution or in semi-liquid form as a gel.

9. The composition according to any one of the preceding claims 1-8, further comprising a pharmacologically active principle selected from antihistamines, antibiotics, anti-glaucomatous drugs, steroidal and non-steroidal anti-inflammatory drugs, antiallergics, antivirals, anesthetics, mydriatics, and mixtures thereof.

10. The composition according to any one of the preceding claims 1-8, wherein hyaluronic acid or a salt thereof is present in an amount comprised in the range from 0.01% to 5%, from 0.05 to 0.5%, from 0.1 to 0.3 w/v, xanthan gum or a salt thereof is comprised in the range from 0.01 to 5%, from 0.05 to 0.5, from 0.1 to 0.3% w/v, glycine is present in an amount comprised in the range from 0.01 to 1 %, from 0.05 to 0.2% w/v and/orbetaine is present in an amount comprised in the range from 0.01 to 3%, from 0.1 to 0.5% w/v.

11. The composition according to any one of the preceding claims 1-10, further comprising mannitol, preferably in an amount comprised in the range from 1.0 to 4.0%.

12. The composition according to any one of the preceding claims 1-11, further comprising bromfenac or a pharmaceutically acceptable salt or ester thereof.

13. The composition according to any one of claims 1-12 for use as a medicament, in particular for use in the treatment of an eye affection or disease in particular in the treatment of back of the eye inflammation, or lesions or abrasions of the ocular surface.

14. The composition according to any one of claims 1-12 for use in the treatment of dry eye.

15. The composition according to any one of claims 1-12, comprising a pharmacologically active principle selected from antihistamines, antibiotics, anti-glaucomatous drugs, steroidal and non-steroidal anti-inflammatory drugs, antiallergics, antivirals, anesthetics, mydriatics and mixtures thereof, for use in the treatment of an eye affection or disease, in particular allergic syndromes, infectious and inflammatory corneal-conjunctival diseases of bacterial or viral origin such as conjunctivitis, blepharitis and keratitis, glaucoma, dry eye with mild-moderate to severe symptoms, and in prevention in cases of ophthalmic surgery.

## Patentansprüche

1. Ophthalmische Zusammensetzung, umfassend ein lineares Polymer auf der Basis von Hyaluronsäure oder ein pharmazeutisch verträgliches Salz davon, ein verzweigtes Polymer auf der Basis von Xanthangummi oder ein pharmazeutisch verträgliches Salz davon und Osmoprotektoren, wobei die Osmoprotektoren eine Kombination aus Glycin und Betain, gegebenenfalls in Form von Salz, vorzugsweise als Betain-HCl, umfassen.

2. Zusammensetzung nach Anspruch 1, wobei Hyaluronsäure oder ein Salz davon in einer Menge vorhanden ist, die im Bereich von 0,01 bis 5 Gew.-%, von 0,05 bis 0,5 Gew.-%, von 0,1 bis 0,3 Gew.-% liegt, und wobei der Xanthangummi oder ein Salz davon in einem Bereich von 0,01 bis 5 Gew.-%, von 0,05 bis 0,5 Gew.-%, von 0,1 bis 0,3 Gew.-% liegt.

3. Zusammensetzung nach Anspruch 1 oder 2, wobei Glycin in einer Menge vorhanden ist, die im Bereich von 0,01 bis 1 Gew.-%, von 0,05 bis 0,2 Gew.-% liegt, und/oder Betain in einer Menge vorhanden ist, die im Bereich von 0,01 bis 3 Gew.-%, von 0,1 bis 0,5 Gew.-% liegt und die Hyaluronsäure gegebenenfalls ein durchschnittliches Molekulargewicht im Bereich von 0,2 MDa bis 2,5 MDa aufweist.

4. Zusammensetzung nach einem der Ansprüche 1-3, wobei die Hyaluronsäure in Form eines pharmazeutisch verträglichen Salzes vorliegt, das aus Natrium, Kalium, Calcium, Magnesium und kationischen Aminosäuren ausgewählt ist.

5. Zusammensetzung nach einem der Ansprüche 1-4, ferner umfassend ein Isotonisierungsmittel, das vorzugsweise aus Natriumchlorid, Kaliumchlorid, Mannitol, Glycerin, Dextrose, Glykol wie beispielweise Propylen- oder Ethylenglykol und Mischungen davon ausgewählt ist.

6. Zusammensetzung nach einem der Ansprüche 1-5, ferner umfassend einen Puffer oder ein Puffersystem, das vorzugsweise aus Acetatpuffern, Citratpuffern, Phosphatpuffern, Boratpuffern, Puffern auf Trometamolbasis, Histidin, Arginin und Puffer- oder Puffersystemmischungen, Emulgatoren, Stabilisatoren, Lösungsvermittlern, Antioxidantien und Mischungen davon ausgewählt ist.

7. Zusammensetzung nach einem der Ansprüche 1-6, die einen pH von 4,0 bis 9,0, vorzugsweise von 6,8 bis 7,6, aufweist.

8. Zusammensetzung nach einem der Ansprüche 1-7 in flüssiger Form als eine wässrige Lösung oder in halbflüssiger Form als ein Gel.

9. Zusammensetzung nach einem der vorhergehenden Ansprüche 1-8, ferner umfassend einen pharmakologischen Wirkstoff, der aus Antihistaminika, Antibiotika, Antiglaukomatosa, steroidalen und nicht-steroidalen entzündungshemmenden Arzneimitteln, Antiallergika, Virostatika, Anästhetika, Mydriatika und Mischungen davon ausgewählt ist.

10. Zusammensetzung nach einem der vorhergehenden Ansprüche 1-8, wobei Hyaluronsäure oder ein Salz davon in einer Menge vorhanden ist, die im Bereich von 0,01 bis 5 Gew.-%, von 0,05 bis 0,5 Gew.-%, von 0,1 bis 0,3 Gew.-% liegt, Xanthangummi oder ein Salz davon in einem Bereich von 0,01 bis 5 Gew.-%, von 0,05 bis 0,5 Gew.-%, von 0,1 bis 0,3 Gew.-% liegt, Glycin in einer Menge vorhanden ist, die im Bereich von 0,01 bis 1 Gew.-%, von 0,05 bis 0,2 Gew.-% liegt, und/oder Betain in einer Menge vorhanden ist, die im Bereich von 0,01 bis 3 Gew.-%, von 0,1 bis 0,5 Gew.-% liegt.

11. Zusammensetzung nach einem der vorhergehenden Ansprüche 1-10, ferner umfassend Mannitol, vorzugsweise in einer Menge, die im Bereich von 1,0 bis 4,0 Gew.-% liegt.

12. Zusammensetzung nach einem der vorhergehenden Ansprüche 1-11, ferner umfassend Bromfenac oder ein pharmazeutisch verträgliches Salz oder einen pharmazeutisch verträglichen Ester davon.

13. Zusammensetzung nach einem der vorhergehenden Ansprüche 1-12 zur Verwendung als Medikament, insbesondere zur Verwendung bei der Behandlung von Augenleiden oder -erkrankungen, insbesondere bei der Behandlung von Entzündungen des Augenhintergrunds oder Läsionen oder Abschürfungen der Augenoberfläche.

14. Zusammensetzung nach einem der Ansprüche 1-12 zur Verwendung bei der Behandlung von trockenen Augen.

15. Zusammensetzung nach einem der Ansprüche 1-12, umfassend einen pharmakologischen Wirkstoff, der aus Antihistaminika, Antibiotika, Antiglaukomatosa, steroidalen und nicht-steroidalen entzündungshemmenden Arzneimitteln, Antiallergika, Virostatika, Anästhetika, Mydriatika und Mischungen davon ausgewählt ist, zur Verwendung bei der Behandlung von Augenleiden oder -erkrankungen, insbesondere bei allergischen Syndromen, infektiösen und entzündlichen Hornhaut-Bindehaut-Erkrankungen bakteriellen oder viralen Ursprungs wie Konjunktivitis, Blepharitis und Keratitis, Glaukom, trockenen Augen mit leichten bis schweren Symptomen und zur Vorbeugung bei augenchirurgischen Eingriffen.

## Revendications

1. Composition ophtalmique comprenant un polymère linéaire à base d'acide hyaluronique ou d'un sel pharmaceutiquement acceptable de celui-ci, un polymère ramifié à base de gomme de xanthane ou d'un sel pharmaceutiquement acceptable de celle-ci, et des osmoprotecteurs, dans laquelle lesdits osmoprotecteurs comprennent une combinaison de glycine et de bétaïne, éventuellement sous forme de sel de préférence comme bétaïne HCl.

2. Composition selon la revendication 1, dans laquelle l'acide hyaluronique ou un sel de celui-ci est présent en une quantité comprise entre 0,01 et 5 %, entre 0,05 et 0,5 %, entre 0,1 et 0,3 p/v, et la gomme de xanthane ou un sel de celle-ci est comprise entre 0,01 et 5 %, entre 0,05 et 0,5 %, entre 0,1 et 0,3 % p/v.

3. Composition selon la revendication 1 ou 2, dans laquelle la glycine est présente en une quantité comprise entre 0,01 et 1 %, entre 0,05 et 0,2 % p/v et/ou la bétaïne est présente en une quantité comprise entre 0,01 et 3 %, entre 0,1 et 0,5 % p/v et éventuellement l'acide hyaluronique a un poids moléculaire moyen compris entre 0,2 MDa et 2,5 MDa.

4. Composition selon l'une quelconque des revendications 1 à 3, dans laquelle l'acide hyaluronique est sous la forme d'un sel pharmaceutiquement acceptable choisi parmi le sodium, le potassium, le calcium, le magnésium et les acides aminés cationiques.

5. Composition selon l'une quelconque des revendications 1 à 4, comprenant en outre un agent d'isotonisation choisi de préférence parmi le chlorure de sodium, le chlorure de potassium, le mannitol, la glycérine, le dextrose, le glycol comme le propylène ou l'éthylène glycol et leurs mélanges.

6. Composition selon l'une quelconque des revendications 1 à 5, comprenant en outre un tampon ou un système tampon choisi de préférence parmi les tampons acétate, les tampons citrate, les tampons phosphate, les tampons borate, les tampons à base de trométamol, l'histidine, l'arginine et les mélanges de tampons ou de systèmes tampons, les agents émulsifiants, les stabilisants, les solubilisants, les antioxydants et leurs mélanges.

7. Composition selon l'une quelconque des revendications 1 à 6, ayant un pH compris entre 4,0 et 9,0, de préférence entre 6,8 et 7,6.

8. Composition selon l'une quelconque des revendications 1 à 7, sous forme liquide comme une solution aqueuse ou sous forme semi-liquide comme un gel.

9. Composition selon l'une quelconque des revendications précédentes 1 à 8, comprenant en outre un principe pharmacologiquement actif choisi parmi les antihistaminiques, les antibiotiques, les médicaments anti-glaucomateux, les médicaments anti-inflammatoires stéroïdiens et non stéroïdiens, les antiallergiques, les antiviraux, les anesthésiques, les mydriatiques et leurs mélanges.

10. Composition selon l'une quelconque des revendications précédentes 1 à 8, dans laquelle l'acide hyaluronique ou un sel de celui-ci est présent en une quantité comprise entre 0,01 % et 5 %, entre 0,05 et 0,5 %, entre 0,1 et 0,3 p/v, la gomme de xanthane, ou un sel de celle-ci est comprise entre 0,01 et 5 %, entre 0,05 et 0,5, entre 0,1 et 0,3 % p/v, la glycine est présente en une quantité comprise entre 0,01 et 1 %, entre 0,05 et 0,2 % p/v et / ou la bétaïne est présente en une quantité comprise entre 0,01 et 3 %, entre 0,1 et 0,5 % p/v.

11. Composition selon l'une quelconque des revendications précédentes 1 à 10, comprenant en outre du mannitol, de préférence en une quantité comprise entre 1,0 et 4,0 %.

12. Composition selon l'une quelconque des revendications précédentes 1 à 11, comprenant en outre du bromfénac ou un sel ou ester pharmaceutiquement acceptable de celui-ci.

13. Composition selon l'une quelconque des revendications 1 à 12, destinée à être utilisée comme médicament, en particulier pour le traitement d'une affection ou d'une maladie oculaire, en particulier dans le traitement de l'inflammation de l'arrière de l'oeil, ou de lésions ou d'abrasions de la surface oculaire.

14. Composition selon l'une quelconque des revendications 1 à 12, destinée à être utilisée dans le traitement de la sécheresse oculaire.

15. Composition selon l'une quelconque des revendications 1 à 12, comprenant un principe pharmacologiquement actif choisi parmi les antihistaminiques, les antibiotiques, les médicaments anti-glaucomateux, les médicaments anti-inflammatoires stéroïdiens et non stéroïdiens, les antiallergiques, les antiviraux, les anesthésiques, les mydriatiques et leurs mélanges, pour le traitement d'une affection ou d'une maladie oculaire, en particulier les syndromes allergiques, les maladies cornéennes-conjonctivales inflammatoires et infectieuses d'origine bactérienne ou virale telles que la conjonctivite, la blépharite et la kératite, le glaucome, la sécheresse oculaire avec des symptômes légers à modérés jusqu'à sévères, et dans la prévention en cas de chirurgie ophtalmique.
